(19) Europäisches Patentamt / European Patent Office / Office européen des brevets

(11) **EP 3 301 181 A1**

(12) **EUROPEAN PATENT APPLICATION**

(43) Date of publication:
**04.04.2018 Bulletin 2018/14**

(51) Int Cl.:
*C12P 5/02* [(2006.01)]    *C12P 7/02* [(2006.01)]
*C12P 7/40* [(2006.01)]    *C12P 7/42* [(2006.01)]
*C12P 7/62* [(2006.01)]    *C12M 1/42* [(2006.01)]

(21) Application number: **16191713.3**

(22) Date of filing: **30.09.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **B.R.A.I.N. Biotechnology Research And Information Network AG 64673 Zwingenberg (DE)**

(72) Inventors:
• **GESCHER, Johannes**
 **76135 Karlsruhe (DE)**
• **REINER, Johannes**
 **76131 Karlsruhe (DE)**
• **MAMPEL, Jörg**
 **64625 Bensheim-Auerbach (DE)**

(74) Representative: **Vossius & Partner Patentanwälte Rechtsanwälte mbB Siebertstrasse 3 81675 München (DE)**

(54) **AUTOTROPHIC MICROBIAL ELECTROSYNTHESIS**

(57) The present application relates to a method for producing a compound comprising reduced carbon by the chemical reduction of a compound comprising carbon having a higher oxidation state as the oxidation state of the reduced carbon within an electrochemical cell, wherein the method comprises (a) culturing autotrophic microorganisms at the cathode of the electrochemical cell in the presence of an average cathode voltage of below +600 mV vs. the standard hydrogen electrode (SHE), wherein the cathode serves as electron donor; (b) contacting the autotrophic microorganisms with $O_2$ and the compound comprising carbon having a higher oxidation state as the oxidation state of the reduced carbon, wherein $O_2$ serves as electron acceptor, said compound serves as the sole carbon source and the knallgas bacteria reduce said compound, thereby producing the compound comprising reduced carbon; and (c) isolating the compound comprising reduced carbon from the electrochemical cell, wherein the method is carried out in the absence of externally added and/or abiotically produced $H_2$ or any other molecular compound externally added and/or abiotically produced as electron donor.

Figure 2

EP 3 301 181 A1

**Description**

The present application relates to a method for producing a compound comprising reduced carbon by the chemical reduction of a compound comprising carbon having a higher oxidation state as the oxidation state of the reduced carbon within an electrochemical cell, wherein the method comprises (a) culturing autotrophic microorganisms at the cathode of the electrochemical cell in the presence of an average cathode voltage of below +600 mV vs. the standard hydrogen electrode (SHE), wherein the cathode serves as electron donor; (b) contacting the autotrophic microorganisms with $O_2$ and the compound comprising carbon having a higher oxidation state as the oxidation state of the reduced carbon, wherein $O_2$ serves as electron acceptor, said compound serves as the sole carbon source and the knallgas bacteria reduce said compound, thereby producing the compound comprising reduced carbon; and (c) isolating the compound comprising reduced carbon from the electrochemical cell, wherein the method is carried out in the absence of externally added and/or abiotically produced $H_2$ or any other molecular compound externally added and/or abiotically produced as electron donor.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Microbial electrosynthesis is a process that was only recently described by Nevin et *al.* as an electricity-driven reduction of carbon dioxide catalyzed by microorganisms (reference 1). The application of this process addresses the demand for an electrical energy storage solution from renewable sources as well as the need for new ways to transform $CO_2$ into valuable compounds (references 1,2,3).

Autotrophic electrosynthesis is a respiratory process in which microbes use the difference in redox potential between a cathode as electron donor and an electron acceptor to establish an ion-gradient that is used for ATP synthesis. The biochemistry that enables the import of electrons from cathodes is so far not well understood and it is still under debate whether electron transfer occurs in all reported cases directly or at least to some extend mediated via cathode driven hydrogen production. Whether or not hydrogen could be produced on the cathode will depend on the choice of the cathode material as well as the working electrode potential. The midpoint redox potential of the $H^+/H_2$ redox couple is -414 mV vs. standard hydrogen electrode (SHE) and Yates et al. could show hydrogen production on graphite block cathodes starting at a potential of -600 mV vs. SHE (reference 4).

The production of bio-based oils by knallgas microorganisms in a reactor vessel is described in WO2013/0990769. This process relies on the addition or production of gaseous electron donors for the cultivation of knallgas organisms. WO2014/043690 describes an electrochemical cell in which hydrogen is produced as electron donor for anaerobic microorganisms that typically produce acetate or methane. Further conversion of these primary products in other useful compounds including the bioplastic poly-3-hydroxy-butyrate (PHB) is conducted in a separate reactor that can contain another electron acceptor besides carbon dioxide. WO2014/198560 shows the use of knallgas-bacteria for the production of useful chemical compounds. The organisms are grown using a gaseous mixture containing the electron donor and electron acceptor as substrate.

The present invention thus aims at providing further means and methods for producing useful compounds (such as bioplastics) from low value carbon sources (such as $CO_2$).

Hence, the present invention relates in a first aspect to a method for producing a compound comprising reduced carbon by the chemical reduction of a compound comprising carbon having a higher oxidation state as the oxidation state of the reduced carbon within an electrochemical cell, wherein the method comprises (a) culturing autotrophic microorganisms at the cathode of the electrochemical cell in the presence of an average cathode voltage of below +600 mV vs. the standard hydrogen electrode (SHE), wherein the cathode serves as electron donor; (b) contacting the autotrophic microorganisms with $O_2$ and the compound comprising carbon having a higher oxidation state as the oxidation state of the reduced carbon, wherein $O_2$ serves as electron acceptor, said compound serves as the sole carbon source and the knallgas bacteria reduce said compound, thereby producing the compound comprising reduced carbon; and (c) isolating the compound comprising reduced carbon from the electrochemical cell, wherein the method is carried out in the absence of externally added and/or abiotically produced $H_2$ or any other molecular compound externally added and/or abiotically produced as electron donor.

In accordance with the present invention a compound comprising carbon (C) is to be produced by reducing another compound comprising carbon. The oxidation state of the carbon in the compound to be produced (i.e. the product) is lower as the oxidation state of the carbon in the compound being the starting compound (i.e. the educt). In accordance with the IUPAC definition the oxidation state is a measure of the degree of oxidation of an atom in a substance. It is defined as the charge of an atom might be imagined to have when electrons are counted according to an agreed-upon set of rules: (1) the oxidation state of a free element (uncombined element) is zero; (2) for a simple (monatomic) ion, the oxidation state is equal to the net charge on the ion; (3) hydrogen has an oxidation state of 1 and oxygen has an oxidation state of -2 when they are present in most compounds (Exceptions to this are that hydrogen has an oxidation state of -1 in hydrides of active metals, e.g. LiH, and oxygen has an oxidation state of -1 in peroxides, e.g. $H_2O_2$); and (4) the algebraic sum of oxidation states of all atoms in a neutral molecule must be zero, while in ions the algebraic sum of the oxidation states of the constituent atoms must be equal to the charge on the ion. The higher the oxidation state of a given atom, the greater is its degree of oxidation; the lower the oxidation state, the greater is its degree of reduction (see IUPAC, Compendium of Chemical Terminology, 2nd ed. (the "Gold Book"), compiled by A. D. McNaught and A. Wilkinson, Blackwell Scientific Publications, Oxford (1997)). Carbon (C) can adopt oxidation states from +IV to -IV. For, example the oxidation state of C is +IV in $CO_2$ while it is - IV in $CH_4$. The increase in oxidation state of an atom is known as an oxidation whereas a decrease in oxidation state is known as a reduction. Oxidations and reductions involve the formal transfer of electrons. While a net gain in electrons is a reduction, a net loss of electrons is an oxidation. The methods described herein involve a reduction. Preferred examples of the chemical reduction that is carried out in the context of the method of the invention are the chemical reduction of a compound comprising carbon having an oxidation state of +IV (C(+IV)) to produce a compound comprising carbon having an oxidation state of +III of less (C($\leq$+III)), and the chemical reduction of a compound comprising carbon having an oxidation state of +III (C(+III)) to produce a compound comprising carbon having an oxidation state of +II of less (C($\leq$+II)).

A compound (or chemical compound) as referred to herein is an entity consisting of two or more atoms, wherein at least two are from different elements which associate via chemical bonds (i.e. preferably via covalent bonds). The compound comprising carbon having a higher oxidation state as the oxidation state of the reduced carbon may be any compound comprising at least one carbon atom having a higher oxidation state as the oxidation state of the reduced carbon. Hence, in case the compound comprises two or more Cs it is sufficient that one such C is comprised. The compound comprising carbon having a higher oxidation state as the oxidation state of the reduced carbon is preferably a C1 compound; i.e. an organic compound that comprises a single carbon atom with attached atoms (such as $CO_2$). Likewise, the compound comprising reduced carbon may be any compound comprising at least one reduced carbon atom (said carbon atom having a lower oxidation state as the oxidation state of the carbon within the compound comprising carbon having a higher oxidation state as the oxidation state of the reduced carbon). The compound comprising reduced carbon is preferably a $\geq$C2 compound; i.e. an organic compound that comprises two or more carbon atoms with attached atoms (such as $CH_3COOH$ acetic acid).

An "electrochemical cell" as used herein defines a device being capable of facilitating or even enabling the reduction reaction of the method of the invention by the introduction of electrical energy, in particular electrons from the cathode. The electrochemical cell comprises a cathode and an anode. A wide range of materials can be used as the material for a cathode and/or anode of an electrochemical cell. For example, the cathode and/or anode can comprise carbon paper, carbon cloth, carbon felt, carbon wool, carbon foam, graphite, porous graphite, graphite powder, graphene, carbon nanotubes, electrospun carbon fibers, a conductive polymer, a metal, and combinations thereof. The material of the base, siding, walls, lining, and/or top of the electrochemical cell is not particularly limited. Examples of suitable materials are bitumen, cement, ceramics, clay, concrete, epoxy, fiberglass, glass, macadam, plastics, sand, sealant, soil, steels or other metals and their alloys, stone, tar, wood, and any combination thereof.

In accordance with the method of the invention the average cathode voltage is below +600 mV vs. the standard hydrogen electrode (SHE). The SHE is the redox electrode which forms the basis of the thermodynamic scale of oxidation-reduction potentials referred to in the context of the claimed method. Its absolute electrode potential is estimated to be 4.44 $\pm$ 0.02 V at 25 °C, but to form a basis for comparison with all other electrode reactions, hydrogen's standard electrode potential ($E^0$) is declared to be zero volts at all temperatures. In the art potentials of any other electrodes are usually compared with that of the SHE at the same temperature. The reference temperature is preferably 25°C. It is of further note that the electron acceptor $O_2$ as used in the method of the invention has a potential of +818 mV vs. SHE, such that cathode voltages below +600 mV generate a sufficient voltage difference (i.e. a voltage difference of at least 218 mV). The average cathode voltage is preferably a constant cathode voltage.

"Autotrophic microorganisms" are microbial organisms capable of synthesizing their own food from inorganic substances, using light or chemical energy. Preferred examples of autotrophic microorganisms are autotrophic bacteria, archae and algae. Most preferred are autotrophic bacteria. The autotrophic microorganisms (or preferably bacteria) are preferably chemotrophic microorganisms (or preferably bacteria) and hence are capable of obtaining chemical energy through oxidation. The autotrophic microorganisms (or preferably bacteria) are more preferably chemolithoautotrophic microorganisms (or preferably bacteria) and hence capable of obtaining energy from the oxidation of inorganic compounds and using a compound comprising carbon (such as carbon dioxide) as its sole source of carbon for growth. The autotrophic microorganisms (or preferably bacteria) are most preferably chemolithoautotrophic microorganisms capable of obtaining energy from the knallgas reaction (which reaction will be further detailed herein below). The autotrophic microorganisms can be comprised within a microbial community with other microorganisms. The autotrophic microorganisms can also be a mixture of different genus/species of autotrophic microorganisms or all autotrophic microorganisms can be of the same genus/species of autotrophic microorganisms.

Means and methods for culturing autotrophic microorganisms at the cathode of an electrochemical cell, including suitable media for culturing the autotrophic microorganisms are described in the context of the examples herein below and available from the prior art, such as WO 2013/090769 and WO 2014/043690. The medium can be removed from the electrochemical cell during the method of the invention periodically or continuously, and the media can be replaced with fresh media. By such measure, for example, the autotrophic microorganisms can be maintained in a high growth phase, or the autotrophic microorganisms can be maintained in a stationary growth phase exhibiting enhanced production of the compound comprising reduced carbon. The medium may be constantly exchanged or periodically exchanged. The autotrophic microorganisms as cultured at the cathode preferably produce a biofilm at the cathode. Within this biofilm the autotrophic microorganisms stick to each other and often may also adhere to a surface of the cathode.

Means and methods for isolating chemical compounds, such as the compound comprising reduced carbon from the electrochemical cell are known in the art. Non-limiting examples are extraction, recrystallization, distillation, and chromatography. Extraction is based on a distinct solubility of the compound to be isolated in a polar solvent (such as $H_2O$) and a nonpolar solvent (such as $CCl_4$). Recrystallization takes advantage of differences in the solubility of various components of a mixture in different solvents. Distillation may, for example, be based on heating up and the distinct boiling point of the compound to be isolated. Distillation generally works best for purifying compounds that are liquids while solids are more likely to be purified by recrystallization. In chromatography a gas or liquid is allowed to flow over a solid support. Compounds that have a high affinity for the solvent are carried along as it moves past the stationary support. Compounds that have a higher affinity for the solid move more slowly.

In the context of the claimed method the cathode serves as the electron ($e^-$) donor and $O_2$ as the electron acceptor when the compound comprising reduced carbon (C) is produced by the chemical reduction of a compound comprising carbon (C) having a higher oxidation state as the oxidation state of the reduced carbon. Hence, in the context of the claimed method the following chemical reaction is carried out:

$$O_2 + n \; x \; e^-$$

Compound comprising C having a higher oxidation state --------> Compound comprising reduced C

This chemical reaction is catalyzed by the autotrophic microorganisms. Hence, it is to be understood that the autotrophic microorganisms are in accordance with the present invention capable of catalyzing the above reaction. The autotrophic microorganisms are furthermore capable of catalyzing the above reaction in the absence of externally added and/or abiotically produced $H_2$ or any other molecular compound externally added and/or abiotically produced as electron donor. In this respect it has to be understood that the autotrophic microorganisms itself might produce $H_2$ or another molecular compound potentially serving as an as electron donor via the electrons from the cathode. The autotrophic microorganisms might also consume said internally or biotically produced $H_2$ or another molecular compound. A molecular compound is a compound in which the elements share electrons via covalent bonds.

It has been surprisingly found in the context of the present invention that autotrophic microorganisms, in particular knallgas bacteria exist that can use electrons originating directly from a cathode as electron acceptor. The cathode is not used for the production of hydrogen but as the direct electron and energy donor for the microorganisms. Hence, the autotrophic microorganisms can catalyze the above chemical reaction in the absence of externally added and/or abiotically produced $H_2$ or any other molecular compound externally added and/or abiotically produced as electron donor. This finding was very unexpected because outside an electrochemical cell autotrophic microorganisms require $H_2$ or another molecular compound as electron donor in order to be capable of producing energy by the reduction of a compound comprising C. However, within an electrochemical cell $H_2$ or another molecular compound as electron donor is no longer needed as an electron donor and these electron donors can unexpectedly be substituted by the electrons of the cathode. Moreover, it was unexpected that even a thin biofilm could sufficiently block the abiotic reduction of oxygen to water at the cathode surface.

Furthermore, and in contrary to WO2014/043690 the electron acceptor of the biocatalysts in the bioelectrochemical system is oxygen. The high redox potential of the $O_2/H_2O$ pair (E°'= 818 mV) allows to sustain the metabolism of the biocatalyst even at high cathode potentials and can lead - according to the Nernst equation - to higher cellular yields per electron transferred. Carbon dioxide is in this case only used as carbon source and not as catabolic electron acceptor. In contrary, the use of carbon dioxide as carbon source and electron acceptor leads to a higher limitation with regard to the applicable cathode potential as the redox potential of the $CO_2$/methane, $CO_2$/acetate, and $CO_2$/formate redox couples is with -244, -290 and -432 mV more than 1 V lower compared to the $O_2/H_2O$ couple.

Therefore, in accordance with a preferred embodiment of the method of the invention the cathode serves as the sole electron donor. No other electron donors are involved in the above described chemical reaction being carried out in the context of the method of the invention.

In accordance with a further preferred embodiment of the method of the invention the autotrophic microorganisms are knallgas bacteria.

Knallgas bacteria (or hydrogen oxidizing bacteria) designate a number of bacterial species that can grow chemolithoautotrophically by obtaining energy from the knallgas reaction. The knallgas reaction is the oxidation of dihydrogen to water by dioxygen. Knallgas bacteria are furthermore capable of producing compounds comprising reduced C from $H_2$ and $CO_2$ in the presence of $O_2$, wherein in this production $H_2$ serves as electron donor and $O_2$ as the electron acceptor. As discussed, it has now been surprisingly found in the context of the present invention that within an electrochemical cell $H_2$ as electron donor is no longer needed as an external electron donor and that this electron donor can unexpectedly be substituted by the electrons of the cathode.

The knallgas bacteria may be naturally occurring (or wild-type) knallgas bacteria or genetically engineered knallgas bacteria. For example, a genetically engineered knallgas bacterium may be E. *coli* into which the genes were introduced that encode the enzymes required for producing compounds comprising reduced C from $H_2$ and $CO_2$ in the presence of $O_2$, wherein in this production $H_2$ serves as electron donor and $O_2$ as the electron acceptor. Genetically engineered knallgas bacteria may also be a genetically modified knallgas bacteria, i.e. a naturally occurring knallgas bacteria into which mutations were introduced, e.g. mutations further modifying the genes encoding the enzymes required for producing compounds comprising reduced C from $H_2$ and $CO_2$ in the presence of $O_2$, wherein in this production $H_2$ serves as electron donor and $O_2$ as the electron acceptor. Means and method for genetic engineering as well as genetic modification are well established in the art. The knallgas bacteria are preferably genetically modified knallgas bacteria or naturally occurring knallgas bacteria and are most preferably naturally occurring knallgas bacteria.

In accordance with a more preferred embodiment of the method of the invention the knallgas bacteria are selected from the species *Achromobacter sp., Acidovorax sp., Acinetobacter sp., Alcaligenes sp., Aquaspirillum sp., Aquifex sp., Bacillus sp., Calderobacterium sp., Corneybacterium sp., Cupriavidus sp., Flavobacterium sp., Helicobacter sp., Hydrogenobacter sp., Hydrogenomonas sp., Hydrogenophaga sp., Hydrogenovibrio sp., Kyrpidia sp., Metallosphaera sp., Mycobacterium sp., Nocardia sp., Paracoccus sp., Pseudomonas sp., Ralstonia sp., Rhodobacter sp., Stenotrophomonas sp., Wautersia sp. and Xanthobacter sp.*

The species of the knallgas bacteria used in the context of the method of the invention is not particularly limited, because it is expected that most knallgas bacteria when cultured at the cathode of an electrochemical cells can use the electrons of the cathode as the electron donor (instead of $H_2$ as in nature). The above species knallgas bacteria are non-limiting examples of suitable knallgas bacteria species. Within this list the species Kyrpidia sp. is most preferred as this species is used in the examples to illustrate the present invention.

In accordance with another preferred embodiment of the method of the invention the compound comprising reduced carbon is a compound comprising carbon having an oxidation state of +III of less (C($\leq$+III)), and wherein the compound comprising carbon having a higher oxidation state as the oxidation state of the reduced carbon is a compound comprising carbon having an oxidation state of +IV (C(+IV)).

In the examples herein below $CO_2$ serves as the educt. 3-hydroxybutyrate is a potential and preferred product. Carbon dioxide ($CO_2$) is a compound comprising carbon having an oxidation state of +IV (C(+IV)) whereas 3-hydroxybutyrate is a compound comprising carbon having an oxidation state of (C($\leq$+III)). Accordingly, in the above preferred embodiment the oxidation state of the carbon in the compound to be produced (i.e. the product) is +III or less (C($\leq$+III)) while the oxidation state of the carbon in the compound being the starting product (i.e. the educt) is +IV (C(+IV)). In this preferred embodiment of the invention the method is consequently for producing a compound comprising carbon having an oxidation state of +III of less (C($\leq$+III)) by the chemical reduction of a compound comprising carbon having an oxidation state of +IV (C(+IV)).

In accordance with a more preferred embodiment of the method of the invention, the compound comprising C(+IV) is selected from $CCl_4$, $CBr_4$, $CI_4$, $CO(Cl)_2$, $CO(Br)_2$, $CO(I)_2$, $NCO^-$, $CH_2CO_2$, $CO_3^{2-}$, and $CO_2$, is preferably $CO_3^{2-}$ or $CO_2$, and is most preferably $CO_2$.

In this list of preferred examples of the compound comprising C(+IV) $CCl_4$, $CBr_4$, $CI_4$, $CO(Cl)_2$, $CO(Br)_2$, $CO(I)_2$, $NCO^-$, $CO_3^{2-}$, and $CO_2$ are all C1 compounds while $CH_2CO_2^-$ is a C2 compound. With respect to $CH_2CO_2^-$ it is of further note that it is a radical. As mentioned above, C1 compounds are generally preferred. With respect to the most preferred compound $CO_2$ it is of note that power-generating stations worldwide release 12 billion tons of carbon dioxide every year as they burn coal, oil or natural gas. Also certain industrial processes and land-use changes emit $CO_2$. Home and commercial heating plants release another 11 billion tons. $CO_2$ is the primary greenhouse gas emitted through human activities and this the driving force of global warming. In 2014, $CO_2$ accounted for about 80.9% of all U.S. greenhouse gas emissions from human activities. Carbon dioxide is naturally present in the atmosphere as part of the Earth's carbon cycle (the natural circulation of carbon among the atmosphere, oceans, soil, plants, and animals). The above described human activities are altering the carbon cycle by adding more $CO_2$ to the atmosphere. Since the Industrial Revolution began around 1750, human activities have contributed substantially to climate change by adding $CO_2$ to the atmosphere. The method of the present invention is particularly suitable to reduce the $CO_2$ pollution of the atmosphere as it foxed the into compounds comprising C($\leq$+III).

In accordance with an even more preferred embodiment of the method of the invention $CO_2$ and $O_2$ are used in the form of a gas comprising 80-99.5% $CO_2$ and 20-0.5% $O_2$. Thus, the method of the present invention allows for the application of exceptionally high oxygen levels.

As mentioned, most of human-made $CO_2$ is produced by power-generating stations and hence in the form of an industrial waste gas. Such gas generally does not solely consist of $CO_2$ but might also comprise $O_2$. The industrial waste gas often comprises 80-99.5% $CO_2$ and 20-0.5% $O_2$. The use of such industrial waste gas is particularly useful in the context of the method of the invention as it comprises sufficient amount $CO_2$ as the carbon source to be reduced and sufficient $O_2$ to serve as the electron acceptor of the reduction.

In accordance with a yet further preferred embodiment of the method of the invention the average cathode voltage is below +500 mV, preferably below +250 mV, more preferably below 0 mV, even more preferably below -150 mV, and most preferably below -250 mV.

This list of cathode voltages with increasing preferences continuously increases the voltage difference with respect to the electron acceptor $O_2$ (+818mV vs. SHE). For an average cathode voltage below +500mV, +250 mV, 0 mV, -150 mV, and -250 mV the voltage difference is at least 300 mV, at least 550 mV, at least 800 mW, at least 950 mV and at least 1050 mV, respectively. Increasing the voltage difference might be a means to speed up the reduction carried out in accordance with the method of the invention. It is furthermore preferred that the average cathode voltage is above -350 mV, and more preferably above -300 mV. Hence, also the following average cathode voltage ranges are envisioned with increasing preference: between +500mV and -350 mV, +250mV and -350 mV, 0mV and -350 mV, -150mV and -350 mV, and -250mV and -350 mV. Preferably, the following average cathode voltage ranges are envisioned with increasing preference: between +500mV and -300 mV, +250mV and -300 mV, 0mV and -300 mV, -150mV and -300 mV, and -250mV and -300 mV.

In accordance with another preferred embodiment of the method of the invention the compound comprising reduced carbon is selected from methane, carboxylate (such as formate, acetate, propionate, butyrate or isobutyrate), hydroxyalkanoate (such as poly-3-hydroxyvalerate, 3-hydroxypropionate or 3-hydroxybutyrate), alcohol (such as methanol, ethanol, propanol, butanol or isobutanol), and organic acids (such as lactic acid, citric acid, gluconic acid and itaconic acid).

The above list of compounds comprising reduced carbon is a non-limiting list of compounds that can be produced by autotrophic microorganisms and in particular by knallgas bacteria. All these compounds are of commercial interest. Methane ($CH_4$) is a biofuel, e.g. for ovens, homes, water heaters, kilns, automobiles, and turbines. Methane may also be used is processes for the synthesis of methanol, acetic acid, or acetic anhydride. Carboxylates are, for example, used in processes for making soaps, detergents, and shampoos. Hydroxyalkanoates are itself bioplastics or can be used for the production of bioplastics. Also alcohols may be used a biofuels. Alcohols are furthermore used as solvents for fats, oils, waxes, perfumes, resins, dyes, gums and hydrocarbons. The most commercially important organic acids are lactic acid, citric acid, gluconic acid and itaconic acid. They are used in a wide variety of applications as flavor enhancers in food, as ingredients in pharmaceuticals, cosmetics and beverages, and also in the chemical industry for the production of biopolymers, coatings, adhesives and others.

In accordance with a more preferred embodiment of the method of the invention the compound comprising reduced carbon is hydroxyalkanoate, preferably 3-hydroxyvalerate, 3-hydroxypropionate or 3-hydroxybutyrate.

As mentioned, in the examples herein below the knallgas bacterium Kyrpidia sp. is used in the examples to illustrate the present invention. Kyrpidia sp. as well as other knallgas bacteria are capable of producing hydroxyalkanoates which can be used for the production of bioplastics. Kyrpidia sp. is in particular capable of producing 3-hydroxybutyrate. For this reason 3-hydroxybutyrate is the most preferred hydroxyalkanoate.

In accordance with an even more preferred embodiment of the method of the invention further comprises after step (b) and before step (c) contacting the compound comprising reduced carbon produced in step (b) with a further microorganism capable of producing a polyhydroxyalkanoate.

In accordance with a most preferred embodiment of the method of the invention the microorganism capable of producing polyhydroxyalkanoate is selected from knallgas bacteria.

Many knallgas bacteria, such as *Kyrpidia sp.* are not only capable of producing hydroxyalkanoates - being the monomers of bioplastics - but also of further processing the hydroxyalkanoate into polyhydroxyalkanoates and hence the bioplastics themselves. Furthermore, microorganisms are known in the art that are not capable of producing hydroxyalkanoates but are only capable of producing hydroxyalkanoates. In the context of the above even more and most preferred embodiments it is thus to be understood that the further microorganism (e.g. knallgas bacterium) used for the production of the polyhydroxyalkanoate can be the same organism as the autotrophic microorganism used for the hydroxyalkanoate or a different microorganism being capable of producing polyhydroxyalkanoate. Also combinations of different microorganisms capable of producing polyhydroxyalkanoate may be used, e.g. in order to influence the nature of the bioplastic to be produced. Preferred examples of microorganisms capable of producing polyhydroxyalkanoate are *Kyrpidia sp., Cupriavidus necator* (also known as *Alcaligenes eutrophus,* or *Ralstonia eutrophus), Methylobacterium rhodesianum, Bacillus megaterium* and combinations thereof, wherein *Kyrpidia sp.* is most preferred.

Preferred examples of polyhydroxyalkanoates are poly-3-hydroxyvalerate, poly-3-hydroxypropionate or poly-3-hydroxybutyrate, wherein poly-3-hydroxybutyrate is most preferred. As mentioned, Kyrpidia sp. is capable of producing poly-3-hydroxybutyrate.

In nature polyhydroxyalkanoates (PHAs) are produced by microorganisms in order to store carbon and energy.

In the method of the invention, the polyhydroxyalkanoates are extracted and isolated from electrochemical cell and microorganisms. More than 150 different monomers can be combined within this family to give materials with extremely different properties. PHAs are more ductile and less elastic than other plastics, and are also biodegradable. These bioplastics are widely used in the medical industry.

In accordance with a preferred embodiment of the method of the invention within the electrochemical cell the cathode is a graphite cathode or a metal cathode.

While the material of the cathode is not particularly limited graphite cathodes or metal cathodes proved as being particularly suitable in the context of the method of the invention. The metal can be platinum, palladium, titanium, gold, silver, nickel, copper, tin, iron, cobalt, tungsten, stainless steel. A graphite cathode is preferred over a metal cathode. Also the material of the cathode is not particularly limited and a platinum anode is preferred as such an anode has been used in the examples herein below.

In accordance with a further preferred embodiment of the method of the invention within the electrochemical cell further comprises a reference electrode.

A reference electrode can be used to monitor the reduction carried out in the context of the method of the invention. A reference electrode is an electrode which has a stable and well-known electrode potential. The high stability of the electrode potential is usually reached by employing a redox system with constant (buffered or saturated) concentrations of each participants of the redox reaction. The reference electrode can be used to measure absolute voltage values. As a reference electrode a standard-calomel-electrode or a silver/silver chloride electrode can be used. The standard-calomel-electrode is a reference electrode based on the reaction between elemental mercury and mercury(I) chloride whereas the silver/silver chloride electrode functions as a redox electrode and the reaction is between the silver metal and the salt silver(I) chloride.

In accordance with another preferred embodiment of the method of the invention the autotrophic microorganisms are cultured such that a biofilm comprising the autotrophic microorganisms is built at the cathode of the electrochemical cell.

The claimed method requires is step (a) culturing the autotrophic microorganisms at the cathode of the electrochemical cell. In this respect it is preferred that the autotrophic microorganisms are cultured such that a biofilm, preferably a thin biofilm comprising the autotrophic microorganisms is built at the cathode of the electrochemical cell. Such a biofilm is particularly advantageous as it can block the abiotic reduction of oxygen to water at the cathode surface. A biofilm is generally a layer of mucilage adhering to a surface and comprising microorganisms. Herein, the microorganisms are autotrophic microorganisms and the surface is the cathode surface. The thin biofilm preferably has a mean thickness of 5 $\mu$m to 1000 $\mu$m, and more preferably of 20 $\mu$m to 500 $\mu$m. Methods for the quantitative analysis of biofilm thickness are available from the art, e.g, Murga et al. (1995), Biotechnol Bioeng; 45(6):503-10.

In accordance with a yet further preferred embodiment of the method of the invention the cathode comprises turnable plates to which a high pressure atmosphere of $CO_2$ and optionally $O_2$ can be applied, wherein the anode comprises shear blades, and wherein the turnable plates can be rotated between the shear blades.

This configuration of the cathode and anode is particularly suitable for producing a compound comprising reduced C by the method of the invention in industrial yields. This is because the cathode provides an optimized growth area for the autotrophic microorganisms and rotation provides the autotrophic microorganisms with an optimized usage of $CO_2$ and optionally $O_2$. In addition, the shear blades allow for controlling the amount of autotrophic microorganisms being cultured at the cathode, such that, for example, a biofilm of a distinct and desired thickness can be maintained. The sheared autotrophic microorganisms can be used for the isolation of isolating the compound comprising reduced carbon.

In this context it is preferred that the shear blades comprise spacers of non-conductive material preventing a short circuit and which spacers mediate the shearing process.

The present invention relates in a second aspect to an electrochemical cell comprising autotrophic microorganisms being cultured at the cathode, wherein the cathode comprises turnable plates to which a high pressure atmosphere of $CO_2$ and optionally $O_2$ can be applied, the anode comprises shear blades, and wherein the turnable plates can be rotated between the shear blades, and which is devoid of externally added hydrogen or any other molecular compound as electron donor.

As described herein above, such an electrochemical cell is particularly suitable for producing a compound comprising reduced C by the method of the invention in industrial yields. It is again preferred that the shear blades comprise spacers of non-conductive material preventing a short circuit and which spacers mediate the shearing process.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show.

**Figure 1:** Development of negative current in the inoculated electrosynthesis reactor (in red) compared to a sterile control (in black). The applied potential was -350 mV vs. SHE. Oxygen was supplied only by the anode reaction and was due to respiratory consumption not detectable in the head space.

**Figure 2:** Development of negative current with an axenic *Kyrpidia spec.* culture (in black) compared to a sterile control (in red). The applied potential was -500 mV vs. SHE. The oxygen concentration in the head space was 0.5%.

The examples illustrate the invention.

**Example 1 - Methods**

Electrochemical culturing conditions

All electrosynthesis experiments were performed in a reactor that was previously described (reference 5). The working electrode chamber of the bioelectrochemical reactor had a volume of 2 L. A 16 $cm^2$ piece of graphite felt (SGL Carbon SE, Germany) served as working electrode (cathode) material and a platinum gauze (0.5 x 5 cm, 1024 mesh/$cm^2$, wire 0.06 mm diameter), inserted into a porous glass tube, was used as the counter electrode (anode). As a reference electrode, a saturated calomel electrode (SCE) (Sensortechnik Meinsberg, Germany) was employed.

For the initial enrichment of thermophilic organisms, an electrosynthesis (ES) minimal media containing 10 mM $NH_4Cl$, 8.7 mM NaCl, 0.3 mM $KH_2PO_4$, 0.1 mM $CaCl_2$ and 0.012 mM $MgSO_4$ supplemented with Wolfe's mineral elixier (DSMZ 792) was used. The reactor was connected to a potentiostat (AUTOLAB, Metrohm, Netherlands) and a constant potential of -594 mV vs. SCE which equals -350 mV vs. SHE was applied. The system was incubated at 60 °C and continuously purged with a mixture of 80% $N_2$ and 20% $CO_2$. Oxygen was constantly produced on the anode. The pH was adjusted to 3.

For the enrichment of mesophilic species a medium containing 0.3 g/L $NH_4Cl$, 0.3 g/L NaCl, 0.1 g/L $CaCl_2$ and 22 ml of a 1 M $NaHCO_3$ solution was employed. The medium was supplemented with trace elements and vitamins. The 1000x concentrated trace element solution contained 190 mg/L $CoCl_2 \cdot 6\ H_2O$, 2 mg/L $CuCl_2 \cdot 2\ H_2O$, 1,1 g/L Fe(II)SO4 $\cdot$ 7 H2O, 0.3 g/L $H_3BO_4$, 3 g/L $Na_2EDTA$, 18 mg/L Na2MoO4 $\cdot$ 2 $H_2O$, 24 mg/L $NiCl_2 \cdot 6\ H_2O$, 42 mg/L $ZnCl_2$. The 1000x concentrated vitamin solution contained 2 mg/L 4-aminobenzoic acid, 1 mg/L biotin, 5 mg/L nicotinic acid, 2.5 mg/L calcium pantothenate, 7.5 mg/L pyrodoxine hydrochloride, 5 mg/L thiamine chloride and 50 mg/L vitamin B12. The pH of the medium was adjusted to 7.

Isolation of distinct organisms

Several strategies were used to obtain pure cultures from the working electrodes. Microbial growth was assessed in the above mentioned mineral media with hydrogen as electron donor and oxygen as electron acceptor. The isolation strategies led so far to six different electroautotrophic microorganisms that all belong to the physiological group of the "knallgas bacteria".

DNA isolation and 16S PCR sequencing

Total DNA was isolated from samples with the innuSPEED Soil DNA kit (Analytic Jena, Germany) according to the manufacturer's instructions. 16S rDNA gene sequences were amplified from the obtained DNA via PCR using iProof polymerase from Biorad (Munich, Germany) with primers specific for bacterial (Bact_341F, CCTACGGGNGGCWGCAG (SEQ ID NO: 1) and Bact_805R, GACTACHVGGGTATCTAATCC (SEQ ID NO: 2); 21) and archaeal (Arch_915F, GTGCTCCCCCGCCAATTCCT (SEQ ID NO: 3) and Uni_1406R, GACGGGCRGTGTGTRCAA (SEQ ID NO: 4)) sequences. The applied thermal cycling program was the following: 98 °C for 2.30 min, followed by 30 cycles of 98 °C for 10 sec, 52 °C (bacteria) or 56 °C (archaea) for 30 sec, 72 °C for 45 sec and a final elongation step at 72 °C for 10 min. The amplified DNA was loaded on an agarose gel for verification of the amplicon sizes. PCR products were purified from the gel using the WIZARD gel purification kit from Promega (Mannheim, Germany) and subsequently sent for sequencing.

## Example 2 - Results

### Electrotrophic growth

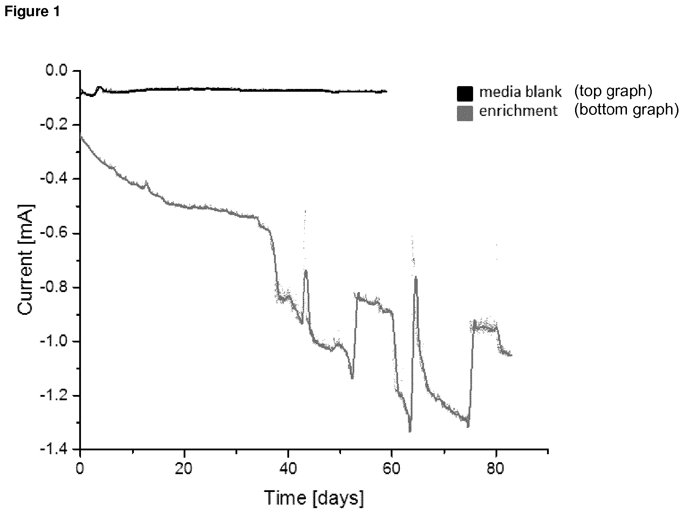

During incubation of different environmental samples a gradual drop in current was observed. To eliminate remaining organics and force a selection for electroautotrophic organisms, the cathodes of the reactors were transferred several times in sterile medium. Figure 1 shows an example of the development of current over time in a bioelectrochemical system. The inoculated reactor reached currents down to 0.75 $A/m^2$ while the current of the sterile control was never lower than 0.063 $A/m^2$ over a period of 60 days. Variations seen in the graph for the inoculated system were due to differences in gassing and media evaporation that was equaled by the addition of $ddH_2O$.

### Electroautotrophic experiments with isolated and enriched organisms

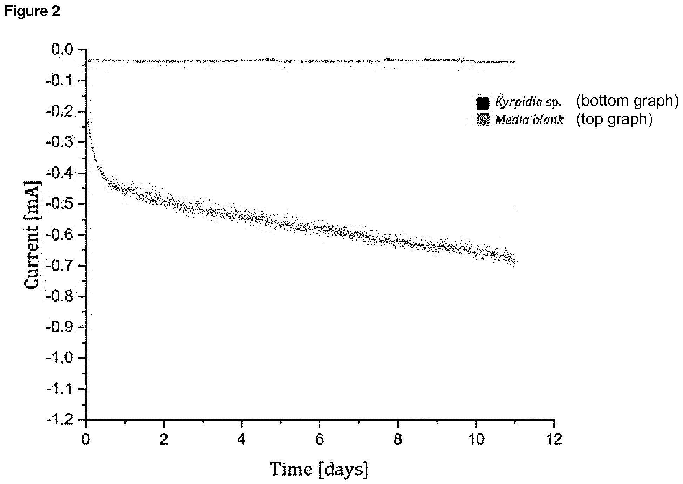

After isolation of individual organism from the cathodes similar experiments in bioelectrochemical systems were conducted with the individual organisms. As an example, the electroautotrophic experiments with the isolated *Kyrpidia* sp. were performed in the media that were described for the isolation of thermophilic species. Sterile medium was used as a control. The applied potential of the working electrode was poised to -500 mV vs. SHE to accelerate growth of the organism. As the isolation of *Kyrpidia* sp. was conducted under microaerophilic conditions a gas mixture of 99.5% $CO_2$ and 0.5% $O_2$ was used in the electroautotrophic experiments. The sterile control reactor never showed currents lower that 0.03 $A/m^2$ whereas a continuous drop in current, due to the consumption of electrons, in the reactor inoculated with *Kyrpidia* sp. was observed throughout the experiment (Fig. 2). By the end of day 11 this was 0.438 $A/m^2$.

## References

1. Nevin KP, Woodard TL and Franks AE, Summers ZM and Lovley DR, Microbial electrosynthesis: feeding microbes electricity to convert carbon dioxide and water to multicarbon extracellular organic compounds. MBio 1:e00103-e00110 (2010)

2. Rabaey K, Girguis P and Nielsen LK, Metabolic and practical considerations on microbial electrosynthesis. Curr Opin Biotechnol 22:371-377 (2011)

3. Lovley DR and Nevin KP, Electrobiocommodities: powering microbial production of fuels and commodity chemicals from carbon dioxide with electricity. Curr Opin Biotechnol 24:385-390 (2013)

4. Yates MD, Siegert N and Logan BE, Hydrogen evolution catalyzed by viable and non-viable cells on biocathodes. International Journal of Hydrogen Energy 39: 16841-16851 (2014)

5. Sturm Richter K, Golitsch F, Sturm G, Kipf E, Dittrich A, Beblawy S, Kerzenmacher S, Gescher J, Unbalanced fermentation of glycerol in Escherichia coli via heterologous production of an electron transport chain and electrode interaction in microbial electrochemical cells. Bioresour Technol. 186:89-96 (2015)

**Claims**

1.  A method for producing a compound comprising reduced carbon by the chemical reduction of a compound comprising carbon having a higher oxidation state as the oxidation state of the reduced carbon within an electrochemical cell, wherein the method comprises

    (a) culturing autotrophic microorganisms at the cathode of the electrochemical cell in the presence of an average cathode voltage of below +600 mV vs. the standard hydrogen electrode (SHE), wherein the cathode serves as electron donor;
    (b) contacting the autotrophic microorganisms with $O_2$ and the compound comprising carbon having a higher oxidation state as the oxidation state of the reduced carbon, wherein $O_2$ serves as electron acceptor, said compound serves as the sole carbon source and the knallgas bacteria reduce said compound, thereby producing the compound comprising reduced carbon; and
    (c) isolating the compound comprising reduced carbon from the electrochemical cell, wherein the method is carried out in the absence of externally added and/or abiotically produced $H_2$ or any other molecular compound externally added and/or abiotically produced as electron donor.

2.  The method of claim 1, wherein the autotrophic microorganisms are knallgas bacteria.

3.  The method of claim 2, wherein the knallgas bacteria are selected from the species *Achromobacter sp., Acidovorax sp., Acinetobacter sp., Alcaligenes sp., Aquaspirillum sp., Aquifex sp., Bacillus sp., Calderobacterium sp., Corneybacterium sp., Cupriavidus sp., Flavobacterium sp., Helicobacter sp., Hydrogenobacter sp., Hydrogenomonas sp., Hydrogenophaga sp., Hydrogenovibrio sp., Kyrpidia sp., Metallosphaera sp., Mycobacterium sp., Nocardia sp., Paracoccus sp., Pseudomonas sp., Ralstonia sp., Rhodobacter sp., Stenotrophomonas sp., Wautersia sp. and Xanthobacter sp.*

4.  The method of any one of claims 1 to 3, wherein the compound comprising reduced carbon is a compound comprising carbon having an oxidation state of +III of less (C($\leq$+III)), and wherein the compound comprising carbon having a higher oxidation state as the oxidation state of the reduced carbon is a compound comprising carbon having an oxidation state of +IV (C(+IV)).

5.  The method of claim 4, wherein the compound comprising C(+IV) is selected from $CCl_4$, $CBr_4$, $Cl_4$, $CO(Cl)_2$, $CO(Br)_2$, $CO(I)_2$, $NCO^-$, $CH_2CO_2^-$, $CO_3^{2-}$, and $CO_2$, is preferably $CH_2CO_2^-$, $CO_3^{2-}$, or $CO_2$, and is most preferably $CO_2$.

6.  The method of claim 5, wherein $CO_2$ and $O_2$ are used in the form of a gas comprising 80-99.5% $CO_2$ and 20-0.5% $O_2$.

7.  The method of any one of claims 1 to 6, wherein the average cathode voltage is below +500mV, preferably below +250 mV, more preferably below 0 mV, even more preferably below -150 mV, and most preferably below -250 mV.

8.  The method of any one of claims 1 to 7, wherein the compound comprising reduced carbon is selected from methane, carboxylate (such as formate, acetate, propionate, butyrate or isobutyrate), hydroxyalkanoate (such as 3-hydroxyvalerate, 3-hydroxypropionate or 3-hydroxybutyrate), alcohol (such as methanol, ethanol, propanol, butanol or isobutanol), and organic acids (such as lactic acid, citric acid, gluconic acid and itaconic acid).

9.  The method of claim 8, wherein the compound comprising reduced carbon is hydroxyalkanoate, preferably 3-hydroxyvalerate, 3-hydroxypropionate or 3-hydroxybutyrate.

10. The method of claim 9, further comprising after step (b) and before step (c) contacting the compound comprising reduced carbon produced in step (b) with a further microorganism capable of producing a polyhydroxyalkanoate.

11. The method of claim 10, wherein the microorganism capable of producing polyhydroxyalkanoate is selected from knallgas bacteria.

12. The method of any one of claims 1 to 11, wherein within the electrochemical cell the cathode is a graphite cathode or a metal cathode.

13. The method of any one of claims 1 to 12, wherein the autotrophic microorganisms are cultured such that a biofilm comprising the autotrophic microorganisms is built at the cathode of the electrochemical cell.

14. The method of any one of claims 1 to 13, wherein the cathode comprises turnable plates to which a high pressure atmosphere of $CO_2$ and optionally $O_2$ can be applied, wherein the anode comprises shear blades, and wherein the turnable plates can be rotated between the shear blades.

15. An electrochemical cell comprising autotrophic microorganisms being cultured at the cathode, wherein the cathode comprises turnable plates to which a high pressure atmosphere of $CO_2$ and optionally $O_2$ can be applied, the anode comprises shear blades, and wherein the turnable plates can be rotated between the shear blades, and which is devoid of externally added hydrogen or any other molecular compound as electron donor.

**Figure 1**

**Figure 2**

Europäisches
Patentamt

European
Patent Office

Office européen
des brevets

# EUROPEAN SEARCH REPORT

**Application Number**

EP 16 19 1713

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/068994 A1 (UNIVERSITY OF QUEENSLAND) 24 June 2010 (2010-06-24)<br>* page 2, line 21 - page 3, line 8 *<br>* page 5, line 1 - page 7, line 2 *<br>* page 14, line 26 - page 16, line 6 *<br>* page 15, lines 10-11 *<br>* pages 18-19; example 1 *<br>* pages 21-25; claims 1-4, 6, 7, 26, 29 *<br>* figure 2 *<br>----- | 1-14 | INV.<br>C12P5/02<br>C12P7/02<br>C12P7/40<br>C12P7/42<br>C12P7/62<br>C12M1/42 |
| A,D | LOVLEY, D.R. & NEVIN, KP.:<br>"Electrobiocommodities: powering microbial production of fuels and commodity chemicals from carbon dioxide with electricity",<br>CURRENT OPINION IN BIOTECHNOLOGY,<br>vol. 24, no. 3, June 2013 (2013-06), pages 385-390, XP002768287,<br>* the whole document *<br>* see especially: *<br>* page 386; figure 1 *<br>* page 387, column 2, line 45 - page 388, column 1, line 22 *<br>----- | 1-14 | |

| | | | TECHNICAL FIELDS SEARCHED (IPC) |
|---|---|---|---|
| | | | C12P<br>C12M |

~~The present search report has been drawn up for all claims~~

| Place of search | Date of completion of the search | Examiner |
|---|---|---|
| Berlin | 17 March 2017 | Fuchs, Ulrike |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-14

☐ The present supplementary European search report has been drawn up for those parts of the European patent application which relate to the invention first mentioned in the claims (Rule 164 (1) EPC).

Europäisches Patentamt

European Patent Office

Office européen des brevets

**LACK OF UNITY OF INVENTION**
**SHEET B**

Application Number

EP 16 19 1713

The Search Division considers that the present European patent application does not comply with the requirements of unity of invention and relates to several inventions or groups of inventions, namely:

1. claims: 1-14

    a method for producing a compound comprising reduced carbon by the chemical reduction of a compound comprising carbon having a higher oxidation state as the oxidation state of the reduced carbon within an electrochemical cell comprising (a) culturing autotrophic microorganisms at the cathode of the electrochemical cell in the presence of an average cathode voltage of below +600 mV vs. the standard hydrogen electrode (SHE), wherein the cathode serves as electron donor, (b) contacting the autotrophic microorganisms with O2 and the compound comprising carbon having a higher oxidation state as the oxidation state of the reduced carbon, wherein O2 serves as electron acceptor, said compound serves as the sole carbon source and the knallgas bacteria reduce said compound, thereby producing the compound comprising reduced carbon, and (c) isolating the compound comprising reduced carbon from the electrochemical cell, wherein the method is carried out in the absence of externally added and/or abiotically produced H2 or any other molecular compound externally added and/or abiotically produced as electron donor

    ---

2. claim: 15

    an electrochemical cell comprising autotrophic microorganisms being cultured at the cathode, wherein the cathode comprises turnable plates to which a high pressure atmosphere of CO2 and optionally O2 can be applied, the anode comprises shear blades, and wherein the turnable plates can be rotated between the shear blades, and which is devoid of externally added hydrogen or any other molecular compound as electron donor

    ---

**EP 3 301 181 A1**

## ANNEX TO THE EUROPEAN SEARCH REPORT
## ON EUROPEAN PATENT APPLICATION NO.

EP 16 19 1713

This annex lists the patent family members relating to the patent documents cited in the above-mentioned European search report.
The members are as contained in the European Patent Office EDP file on
The European Patent Office is in no way liable for these particulars which are merely given for the purpose of information.

17-03-2017

| Patent document cited in search report | Publication date | Patent family member(s) | Publication date |
|---|---|---|---|
| WO 2010068994 A1 | 24-06-2010 | AU 2009328649 A1<br>CA 2747212 A1<br>CN 102282295 A<br>EP 2373832 A1<br>JP 2012512326 A<br>US 2011315560 A1<br>WO 2010068994 A1 | 28-07-2011<br>24-06-2010<br>14-12-2011<br>12-10-2011<br>31-05-2012<br>29-12-2011<br>24-06-2010 |

EPO FORM P0459

For more details about this annex : see Official Journal of the European Patent Office, No. 12/82

18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 20130990769 A
- WO 2014043690 A
- WO 2014198560 A
- WO 2013090769 A

### Non-patent literature cited in the description

- Compendium of Chemical Terminology. Blackwell Scientific Publications, 1997
- **MURGA et al.** *Biotechnol Bioeng,* 1995, vol. 45 (6), 503-10
- **NEVIN KP ; WOODARD TL ; FRANKS AE ; SUMMERS ZM ; LOVLEY DR.** Microbial electrosynthesis: feeding microbes electricity to convert carbon dioxide and water to multicarbon extracellular organic compounds. *MBio,* 2010, vol. 1, e00103-e00110
- **RABAEY K ; GIRGUIS P ; NIELSEN LK.** Metabolic and practical considerations on microbial electrosynthesis. *Curr Opin Biotechnol,* 2011, vol. 22, 371-377
- **LOVLEY DR ; NEVIN KP.** Electrobiocommodities: powering microbial production of fuels and commodity chemicals from carbon dioxide with electricity. *Curr Opin Biotechnol,* 2013, vol. 24, 385-390
- **YATES MD ; SIEGERT N ; LOGAN BE.** Hydrogen evolution catalyzed by viable and non-viable cells on biocathodes. *International Journal of Hydrogen Energy,* 2014, vol. 39, 16841-16851
- **STURM RICHTER K ; GOLITSCH F ; STURM G ; KIPF E ; DITTRICH A ; BEBLAWY S ; KERZENMACHER S ; GESCHER J.** Unbalanced fermentation of glycerol in Escherichia coli via heterologous production of an electron transport chain and electrode interaction in microbial electrochemical cells. *Bioresour Technol.,* 2015, vol. 186, 89-96

SEQUENCE LISTING

<110> B.R.A.I.N. Biotechnology Research And Information Network AG

<120> Autotrophic microorganisms for the production of reduced chemical compounds

<130> Z1902 EP

<160> 4

<170> BiSSAP 1.3

<210> 1
<211> 17
<212> PRT
<213> Artificial Sequence

<220>
<223> Bact 341F

<400> 1
Cys Cys Thr Ala Cys Gly Gly Gly Asn Gly Gly Cys Trp Gly Cys Ala
1               5                   10                  15
Gly


<210> 2
<211> 21
<212> PRT
<213> Artificial Sequence

<220>
<223> Bact 805R

<400> 2
Gly Ala Cys Thr Ala Cys His Val Gly Gly Gly Thr Ala Thr Cys Thr
1               5                   10                  15
Ala Ala Thr Cys Cys
            20

<210> 3
<211> 20
<212> PRT
<213> Artificial Sequence

<220>
<223> Arch 915F

<400> 3
Gly Thr Gly Cys Thr Cys Cys Cys Cys Cys Gly Cys Cys Ala Ala Thr
1               5                   10                  15
Thr Cys Cys Thr
            20

<210> 4
<211> 18
<212> PRT
<213> Artificial Sequence

<220>
<223> Uni 1406R

```
<400> 4
Gly Ala Cys Gly Gly Gly Cys Arg Gly Thr Gly Thr Gly Thr Arg Cys
1               5                   10                      15
Ala Ala
```